# EUROPEAN PATENT APPLICATION

(11) **EP 1 969 991 A2**
(43) Date of publication of application: **17.09.2008**
(21) Application number: 08001533.2
(22) Date of filing: 28.01.2008
(51) Int. Cl.: A61B 1/01, A61B 1/31

(54) **Endoscope advancing apparatus**

(30) Priority: 14.03.2007 JP 2007065659
(71) Applicant: Fujinon Corporation, Saitama-shi, Saitama (JP)
(72) Inventor: Fujikura, Tetsuya, Saitama-shi Saitama (JP)
(74) Representative: Klunker . Schmitt-Nilson . Hirsch

(57) **Abstract**

An endoscope apparatus comprises: an endoscope including an insertion section; a cylindrical insertion assisting tool that allows the insertion section to be inserted to assist insertion of the insertion section into a body cavity; and a ring-shaped seal member that is supported so as to slide on the insertion section with the insertion section inserted, wherein the seal member is detachably mounted on a base end of the insertion assisting tool to seal a gap between the insertion assisting tool and the insertion section.

## Description

### Background of the Invention

### 1. Field of the Invention

The present invention relates to an endoscope apparatus, and more particularly to the endoscope apparatus that observes a deep alimentary canal such as a small intestine or a large intestine.

### 2. Description of the Related Art

Since a deep alimentary canal such as a small intestine or a large intestine is complicatedly wound, it is difficult to transmit a force to the front end of an insertion section just by pushing the insertion section of an endoscope. Accordingly, insertion into the deep part of the alimentary canal is difficult. For this reason, there has been proposed a method of inserting the insertion section of the endoscope into a body cavity by inserting the insertion section thereof into a cylindrical insertion assisting tool (which is also called an over-tube or a sliding tube). According to this method, since the insertion section is guided to the insertion assisting tool, it is possible to prevent the insertion section from being bent, thereby inserting the insertion section into the deeper part of the alimentary canal.

In JP-A-10-248794, there is disclosed an endoscope apparatus in which a first balloon is provided on the front end of the insertion section of the endoscope and a second balloon is provided on the front end of the insertion assisting tool. In the endoscope apparatus, the insertion section or the insertion assisting tool can be fixed to the alimentary canal by inflating the first balloon or the second balloon. Accordingly, it is possible to insert the insertion section into the deep part of the alimentary canal while inflating and deflating the corresponding balloon to alternately insert the insertion section and the insertion assisting tool.

However, in the endoscope apparatus using the insertion assisting tool, a body fluid may flow backward beyond a gap between the inner circumferential surface of the insertion assisting tool and the outer circumferential surface of the insertion section. Accordingly, a problem arises in that the body fluid may leak from the base end of the insertion assisting tool and makes the periphery dirty. In particular, in an endoscope apparatus using double balloon, the insertion section is repeatedly inserted into and taken out from the insertion assisting tool. Accordingly, a problem arises in that the body fluid adhering on the insertion section may easily leak from the base end of the insertion assisting tool during insertion of the insertion section.

In order to solve the above-described problems, there has been a method of providing a meal member on the base end of the insertion assisting tool to seal the gap between the insertion assisting tool and the insertion section of the endoscope. In Fig. 22 of JP-A-2005-312905, for example, there is provided a substantially cylindrical tube formed of an elastic body and disclosed in a method of mounting one end of the tube on the base end of the insertion assisting tool and closely contacting the other end of the tube with the outer circumferential surface of the insertion section to seal the gap between the insertion assisting tool and the insertion section.

However, in JP-A-2005-312905, there is a problem in that when the insertion section is inserted into the deep part of the alimentary canal by an insertion operation and an extraction operation, it is difficult to obtain good operability. That is, in order to completely prevent the leakage of the fluid, adhesion between the tube and the insertion section is necessary to improve. However, in this case, since resistance increases at the time the insertion assisting tool and the insertion section are relatively moved by the insertion operation and the extraction operation, an operator cannot directly feel the resistance occurring between the alimentary canal and the insertion assisting tool or between the alimentary canal and the insertion section. As a result, a problem arises in that the operability deteriorates.

### Summary of the Invention

The invention is conceived in view of the above-described problems and the object of the invention is to provide an endoscope apparatus capable of reliably preventing leakage of the body fluid from the base end of the insertion assisting tool and having good operability.

According to Aspect 1 of the invention, there is provided an endoscope apparatus comprising: an endoscope including an insertion section; a cylindrical insertion assisting tool that allows the insertion section to be inserted to assist insertion of the insertion section into a body cavity; and a ring-shaped seal member that is supported so as to slide on the insertion section with the insertion section inserted, wherein the seal member is detachably mounted on a base end of the insertion assisting tool to seal a gap between the insertion assisting tool and the insertion section.

According to Aspect 1 of the invention, the seal member can follow the insertion section in the state where the insertion section is inserted into the seal member. Accordingly, since the seal member can be mounted on the insertion assisting tool if necessary, it is possible to prevent the leakage of the body fluid or the like and to also improve the operability. For example, when the insertion section of the endoscope is inserted into the winding portion of the alimentary canal, it is possible to feel the resistance of the alimentary canal by extracting the seal member from the insertion assisting tool. Moreover, when the insertion section of the endoscope is inserted into the deep part of the alimentary canal, it is possible to prevent the leakage of the body fluid by mounting the sealing member on the insertion assisting tool. In this way, it is possible to improve the operability and to prevent the leakage of the body fluid or the like.

The invention described in Aspect 2 is the endoscope apparatus according to Aspect 1, wherein the seal member comprises: an elastic member including a cylindrical portion having a uniform diameter, an inner circumferential surface of the cylindrical portion being to closely contact an outer circumferential surface of the insertion section; and a ring-shaped tubular body of a material harder than that of the elastic member that supports the elastic member and is detachably fitted to the base end of the insertion assisting tool.

According to Aspect 2 of the invention, the elastic member has the cylindrical portion with the uniform diameter and the inner circumferential surface of the cylindrical portion closely contacts the outer circumferential surface of the insertion section. Accordingly, the surface of the seal member contacts that of the insertion section. Accordingly, since the gap between the insertion assisting tool and the insertion section, it is possible to reliably prevent the leakage of the body fluid or the like.

According to Aspect 2 of the invention, the elastic member is supported by the hard tubular body. Accordingly, the seal member can be easily moved by gripping the tubular body. Moreover, since the tubular body is fitted to the base end of the insertion assisting tool, the seal member can be easily attached to or detached from the insertion assisting tool.

The cylindrical portion of the elastic member may have a predetermined length in an axial direction thereof (for example, in the range of 10 to 20 mm).

The invention described in Aspect 3 is the endoscope apparatus according to Aspect 2, wherein when the tubular body is fitted to the base end of the insertion assisting tool, the elastic member closely contacts the base end of the insertion assisting tool. According to Aspect 3 of the invention, since the elastic member closely contacts the base end of the insertion assisting tool, the elastic member can seal the gap between the insertion assisting tool and the elastic member.

The invention described in Aspect 4 is the endoscope apparatus according to any one of Aspect 2 or 3, wherein the tubular body comprises a locking portion protruding from the inner circumferential surface of the tubular body, and wherein the locking portion is fitted to the base end of the insertion assisting tool. According to Aspect 4, the seal member can be reliably mounted on the base end of the insertion assisting tool by use of the locking portion.

The invention described in Aspect 5 is the endoscope apparatus according to any one of Aspect 2 or 3, wherein the seal member further comprises a mounting ring having a diameter larger than that of the elastic member and having a length in an axial direction shorter than that of the elastic member, and wherein both ends of the elastic member are turned over in a state where the elastic member is inserted into the mounting ring and adhered to an outer circumferential surface of the mounting ring, so that the mounting ring is inserted into the tubular body.

### Brief Description of the Drawings

Fig. 1 is a diagram illustrating a system configuration of an endoscope apparatus according to the invention;
Fig. 2 is perspective view illustrating the front end of an insertion section of an endoscope;
Fig. 3 is a perspective view illustrating a gripping section of an insertion assisting tool and a seal member;
Fig. 4 is a vertical sectional view illustrating the seal member;
Figs. 5A and 5B are diagrams illustrating the assembled seal member;
Figs. 6A to 6J are diagrams for explaining a method of operating the endoscope apparatus shown in Fig. 1;
Figs. 7A and 7B are diagrams for explaining a method of using the seal member; and
Fig. 8 is a vertical sectional view illustrating a seal member with a shape different from that in Fig. 4.

### Detailed Description of the Invention

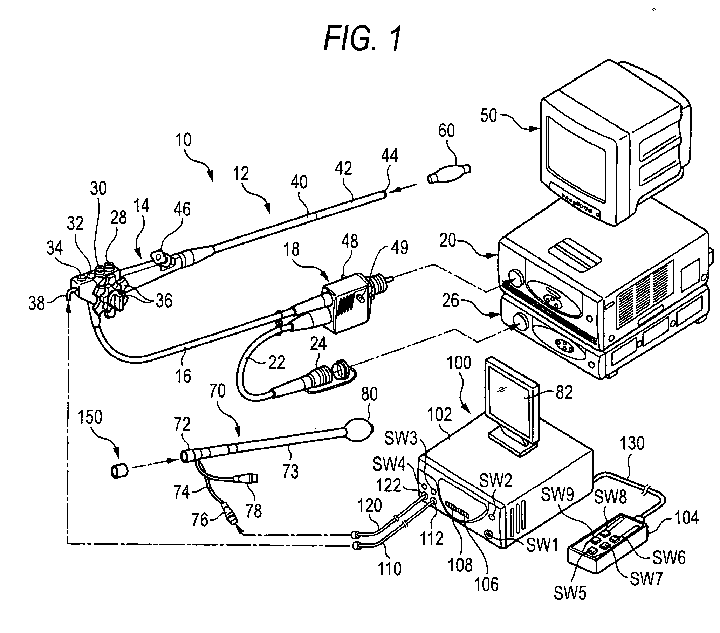

An endoscope apparatus according to an exemplary embodiment of the invention will be described in detail with reference to the accompanying drawings. Fig. 1 is a diagram illustrating a configuration of a system which is an example of the endoscope apparatus according to the invention. As shown in Fig. 1, the endoscope apparatus mainly includes an endoscope 10, an insertion assisting tool 70, a balloon control device 100, and a seal member 150.

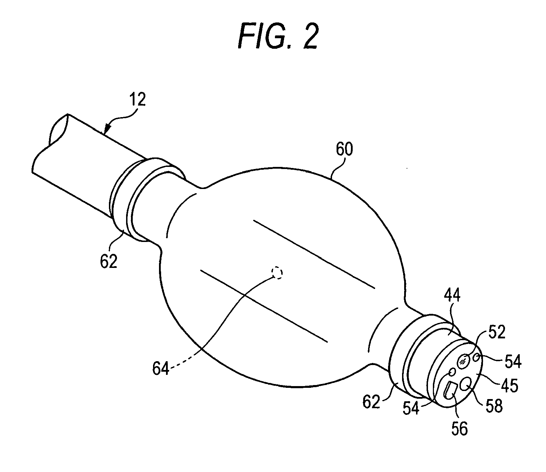

The endoscope 10 includes a hand operation section 14 and an insertion section 12 which is connected to the hand operation section 14 and inserted into a body cavity. The hand operation section 14 extends to a universal cable 16 and an LG connector 18 is provided in the front end of the universal cable 16. The LG connector 18 is connected to a light source device 20 so as to be freely attached thereto or detached therefrom, so that illuminating light is sent to illumination optical systems 54 (see Fig. 2) described below. In addition, an electrical connector 24 is connected to the LG connector 18 through a cable 22, and the electrical connector 24 is connected to a processor 26 so as to be freely attached thereto or detached therefrom.

In the hand operation section 14, an air/water supply button 28, a suction button 30, a shutter button 32, and a function switch button 34 are disposed in parallel and a pair of angle knobs 36 are disposed. A balloon air supply port 38 which is formed of a tube bent in an L shape is formed on the base end of the hand operation section 14. A first balloon 60 described below can be inflated or deflated by supplying a fluid such as air to the balloon air supply port 38 or sucking the fluid from the balloon air supply port 38.

The insertion section 12 includes a flexible portion 40, a curving portion 42, and a front end portion 44 which are sequentially disposed from a side of the hand operation section 14. The curving portion 42 is remotely operated to be curved by rotational operation of the angle knobs 36 disposed on the hand operation section 14. Thereby, the front end portion 44 can be directed in a desired direction.

As shown in Fig. 2, an observation optical system 52, the illumination optical systems 54, an air/water supply nozzle 56, and forceps port 58 are formed on a tip end surface 45 of the front end portion 44. A CCD (not shown) is disposed on the rear side of the observation optical system 52 and a signal cable (not shown) is connected to a board for supporting the CCD. The signal cable is inserted through the insertion section 12, the hand operation section 14, and the universal cable 16, and the like shown in Fig. 1 to extend to the electric connector 24 and the processor 26. Consequently, an image observed by the observation optical system 52 is formed on a light acceptance surface of the CCD to be converted into an electrical signal and the electrical signal is output to the processor 26 through the signal cable to be converted into an image signal. In this way, the observed image is displayed on a monitor 50 connected to the processor 26.

A light-emitting of a light guide (not shown) is disposed on the rear side of the illumination optical systems 54 shown in Fig. 2. The light guide is inserted through the insertion section 12, the hand operation section 14, and the universal cable 16 shown in Fig. 1 so that the light incident end is disposed in the inside of the LG connector 18. Accordingly, the LG connector 18 is connected to the light source device 20, so that illumination light from the light source device 20 is transmitted to the illumination optical systems 54 through the light guide to be radiated from the illumination optical systems 54 to the front side through the light guide.

The air/water supply nozzle 56 shown in Fig. 2 communicates with a valve (not shown) operated by the air/water supply button 28 shown in Fig. 1. In addition, the valve communicates with the air/water supply connector 48 disposed on the LG connector 18. The air/water supply connector 48 is connected to an air/water supply mechanism (not shown) so that air or water is supplied thereto. Accordingly, air or water can be ejected from the air/water supply nozzle 56 to the observation optical system 52 by operation of the air/water supply button 28.

The forceps port 58 shown in Fig. 2 communicates with a forceps insertion portion 46 shown in Fig. 1. Accordingly, a treatment tool such as a forceps is inserted from the forceps insertion portion 46 so as to be taken out from the forceps port 58. The forceps port 58 communicates with the valve (not shown) operated by the suction button 30. In addition, the valve is connected to a suction connector 49 of the LG connector 18. Accordingly, a suction mechanism (not shown) is connected to the suction connector 49, so that a lesion part or the like can be sucked through the forceps port 58 by operating the valve with the suction button 30.

A first balloon 60 made of an elastic material such as rubber is mounted on the outer circumferential surface of the insertion section 12. Both shrinkable ends of the first balloon 60 are formed in a substantially cylindrical shape. The insertion section 12 is inserted into the first balloon 60 so that the first balloon 60 is located at a desired position. Afterward, as shown in Fig. 2, fixation rings 62 made of a rubber material are inserted on both the ends of the first balloon 60 so that the first balloon 60 is fixed to the insertion section 12.

An airing hole 64 is formed at a position where the first balloon 60 is mounted, that is, on the outer circumferential surface of the insertion section 12. The airing hole 64 communicates with the balloon air supply port 38 formed in the hand operation section 14 shown in Fig. 1. In addition, the balloon air supply port 38 is connected to a balloon control device 100 through a tube 110 described below. Accordingly, the first balloon 60 can be inflated or deflated by supplying or sucking air by use of the balloon control device 100. In addition, by supplying air to the first balloon 60, the first balloon 60 is inflated in a substantially spherical shape, and by sucking air, the first balloon 60 is attached to the outer surface of the insertion section 12.

Meanwhile, the insertion assisting tool 70 shown in Fig. 1 includes a hard gripping section 72 which is formed in a cylindrical shape and provided on the base end and a main body tube 73 which is provided on the front end of the gripping section 72. A seal member 150 described below is detachably mounted on the gripping section 72. In addition, the insertion section 12 of the endoscope 10 is inserted in the inside of the main body tube 73 through the gripping section 72.

An inlet port 78 is formed on the base end of the insertion assisting tool 70. The inlet port 78 communicates with an opening (not shown) formed in the inner circumferential surface of the insertion assisting tool 70. Accordingly, a lubricant can be supplied to the inside of the insertion assisting tool 70 by injecting the lubricant (for example, water) from the inlet port 78 with an injector or the like. When the insertion section 12 is inserted into the insertion assisting tool 70, it is possible to reduce friction between the inner circumferential surface of the insertion assisting tool 70 and the outer circumferential surface of the insertion section 12. Accordingly, smoothly relative movement between the insertion section 12 and the insertion assisting tool 70 is possible.

The main body tube 73 of the insertion assisting tool 70 is formed of a flexible resin tube made of urethane or the like, of which the outer circumferential surface and the inner circumferential surface are coated with a hydrophilic coating material (lubricating coating material). As the hydrophilic coating material, for example, polyvinylpyrrolidone is used.

A second balloon 80 is mounted in the vicinity of the front end of the main body tube 73. The second balloon 80 is formed in a substantially cylindrical shape so that both ends thereof are narrowed. The second balloon 80 is fixed by winding a thread (not shown) in a state where the insertion assisting tool 70 is inserted. A tube 74 attached on the outer circumferential surface of the insertion assisting tool 70 communicates with the second balloon 80 and a connector 76 is provided on the base end of the tube 74. A tube 120 is connected to the connector 76 which is connected to the balloon control device 100 through the tube 120. Accordingly, the second balloon 80 can be inflated or deflated by supplying air or sucking air with the balloon control device 100. By supplying air to the second balloon 80, the second balloon 80 is inflated in a substantially spherical shape to be attached to the outer surface of the insertion assisting tool 70 by sucking air.

The balloon control device 100 shown in Fig. 1 is a device which not only supplies a fluid such as air to the first balloon 60 or sucks the fluid from the first balloon 60, but also supplies a fluid such as air to the second balloon 80 or sucks the fluid from the second balloon 80. The balloon control device 100 mainly includes a device body 102 and a hand switch 104 for a remote control.

A power supply switch SW1, a stop switch SW2, a first pressure display unit 106, a second pressure display unit 108, a first function stop switch SW3, and a second function stop switch SW4 are formed on the front surface of the device body 102. The first pressure display unit 106 and the second pressure display unit 108 are panels for displaying a pressure difference of the first balloon 60 and a second balloon 80, respectively. If a problem such as a case where the balloon is torn occurs, an error code is displayed on the pressure display units 106 and 108.

The first function stop switch SW3 and the second function stop switch SW4 are switches for turning ON/OFF a function for a control system of the endoscope and a function for a control system of the insertion assisting tool, respectively. When one of the first balloon 60 and the second balloon 80 is not used, the function is turned OFF by operation of the corresponding one of the function stop switch SW3 and the function stop switch SW4. In the control system in which the function is turned OFF, the supply or suction of air completely stops, and thus the corresponding one of the pressure display unit 106 and the pressure display unit 108 is also turned OFF. When both the function stop switches SW3 and SW4 are tuned OFF, an initial setting process or the like can be performed. For example, a calibration for atmosphere pressure is performed by turning both the function stop switches SW3 and SW4 OFF and simultaneously pressing all switches SW5 to SW9 of the hand switch 104.

A tube 110 for supplying air to the first balloon 60 or sucking air from the first balloon 60 and a tube 120 for supplying air to the second balloon 80 or sucking air from the second balloon 80 are connected to the front surface of the device body 102. Backflow prevention units 112 and 122 are disposed in connection portions between the tubes 110 and 120 and the device body 102, respectively, in order to prevent the backflow of the body liquid in a case where a body fluid flows backward when the first balloon 60 or the second balloon 80 is torn. The backflow prevention units 112 and 122 are configured in a manner gas-liquid separating filter is inserted into a hollow disk-shaped case (not shown) mounted so as to be freely attached to or detached from the device body 102. In this way, the filter can prevent the body fluid from flowing in the device body 102.

The stop switch SW5 which is the same as the top switch SW2 of the device body 102, the ON/OFF switch SW6 for instructing pressurization or depressurization of the first balloon 60, the pose switch SW7 for maintaining the pressure of the first balloon 60, the ON/OFF switch SW8 for instructing pressurization or depressurization of the second balloon 80, and the pose switch SW9 for maintaining the pressure of the second balloon 80 are formed in the hand switch 104. In addition, the hand switch 104 is electrically connected to the device body 102 through a code 130. In the hand switch 104, there is provided a display unit (not shown in Fig. 1) which displays a state of air supply or air discharge of the first balloon 60 or the second balloon 80.

The balloon control device 100 configured in such a manner supplies air to expand the first balloon 60 or the second balloon 80 and also controls the air pressure at a uniform value to maintain the inflated state of the first balloon 60 or the second balloon 80. In addition, the balloon control device 100 sucks the air from the first balloon 60 or the second balloon 80 to contract the first balloon 60 or the second balloon 80 and also controls the air pressure at a uniform value to maintain the deflated state of the first balloon 60 or the second balloon 80.

The balloon control device 100 is connected to a balloon-only monitor 82. In addition, when the balloon control device 100 expands or contracts the first balloon 60 or the second balloon 80, the balloon control device 100 displays the pressure value or the inflated or deflated state of the first balloon 60 or the second balloon 80 on the balloon-only monitor 82. Moreover, the inflated or deflated state of the first balloon 60 or the second balloon 80 may be superimposed on the image observed in the endoscope 10 to be displayed on a monitor 50.

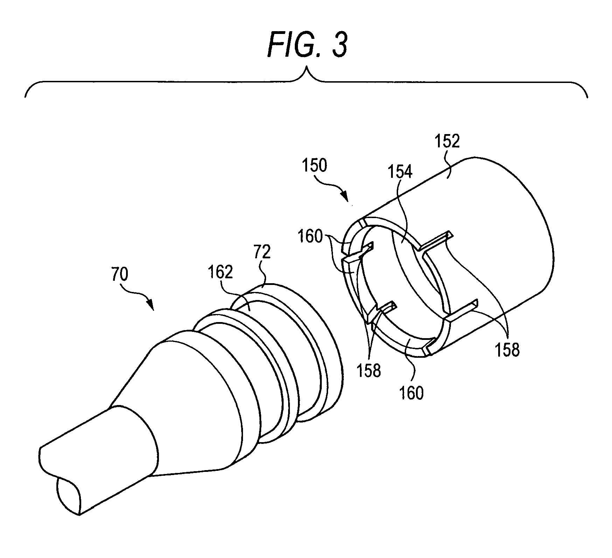

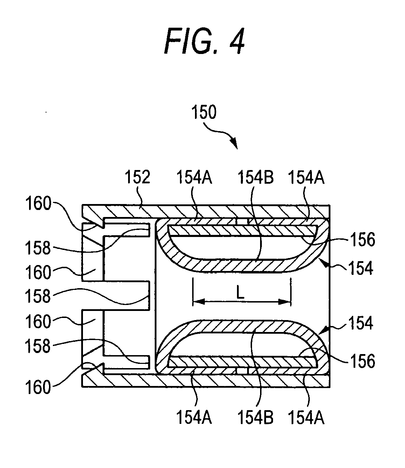

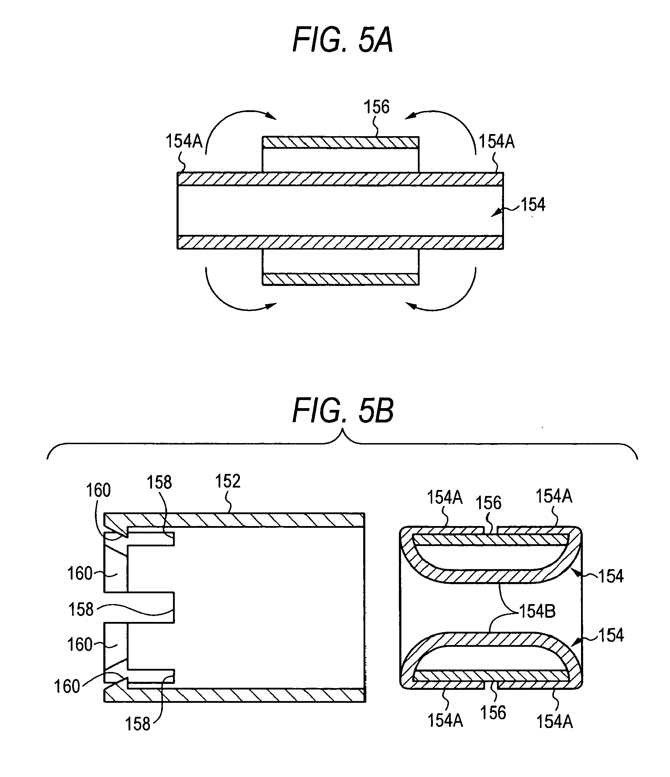

Next, the seal member 150, which is an advantageous aspect of the invention, will be described with reference to Figs. 3 to 5. Fig. 3 is a perspective view illustrating the gripping section 72 of the insertion assisting tool 70 and the seal member 150. Fig. 4 is a longitudinal sectional view illustrating the seal member 150. Figs. 5A and 5B are diagrams illustrating the assembled seal member 150.

As shown in Figs. 3 to 5B, the seal member 150 includes a tubular body 152, an elastic member 154, and a mounting ring 156.

The elastic member 154 is supported on the tubular body 152 through the mounting ring 156. The elastic member 154 is formed of an elastic material such as crude rubber or silicon rubber. In addition, the elastic member 154 is formed in a cylindrical shape (tube shape) with a uniform diameter, as shown in Fig. 5A. The inner diameter of the elastic member 154 is slightly smaller than the outer diameter of the insertion section 12 of the endoscope 10. On the other hand, the diameter of the mounting ring 156 is larger than the outer diameter of the insertion section 12 of the outer diameter of the elastic member 154. In addition, the length of the mounting ring 156 with a cylindrical shape is shorter than that of the elastic member 154 in an axial direction thereof. The elastic member 154 is inserted into the mounting ring 156, and then both the ends 154A of the elastic member 154 are turned over to be fixed to the outer surface of the mounting ring 156 as shown in Fig. 5B. As shown in Fig. 4, the elastic member 154 is supported by the tubular body 152 by fitting the mounting ring 156 mounting with the elastic member 154 to the inside of the tubular body 152. The elastic member 154 supported in this manner is formed so as to have a uniform diameter and has a cylindrical portion 154B with a predetermined length L (for example, in the range of 10 to 20 mm) in an axial direction thereof.

The tubular body 152 made of a material, for example, a resin or metal harder than the elastic member 154 is formed in a ring shape. A plurality of slits 158 are formed from one end surface of the tubular body 152. The slits 158 are formed at a uniform angle interval in a circumferential direction of the tubular body 152. The formation of the slits 158 enables the diameter of the end of the tubular body 152 to be expanded. Claw-like portions (locking portions) 160, which are fitted to the gripping section 72 of the insertion assisting tool 70, are formed on the end of the tubular body 152. The claw-like portions 160 are formed between the slits 158 so as to protrude toward the inner circumferential surface of the tubular body 152. The claw-like portions 160 are engaged with a groove 162 formed around the outer circumference of the gripping section 72. Accordingly, the diameter of the end of the tubular body 152 is slightly expanded and by pushing the tubular body 152 to the gripping section 72 of the insertion assisting tool 70 from the end with the claw-like portions 160 of the tubular body 152. At this time, the claw-like portions 160 are fitted to the groove 162 of the gripping section 72, so that the tubular body 152 is fixed to the gripping section 72.

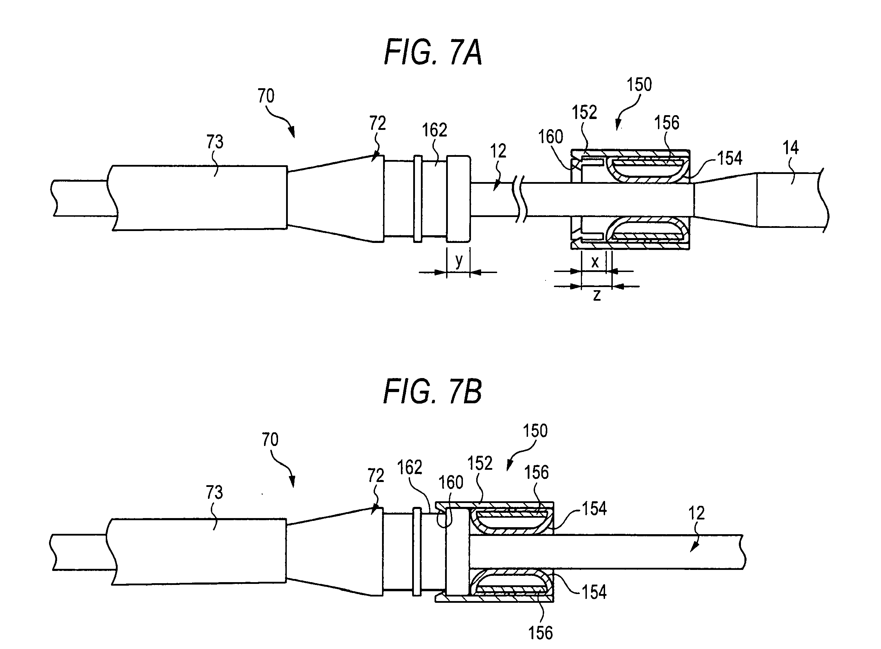

When the tubular body 152 is fixed to the gripping section 72 in this manner, the elastic 154 is configured to be pressed between the mounting ring 156 and the base end of the insertion assisting tool 70 (see Fig. 7B). That is, in Fig. 7A, if it is supposed that a distance between the base end of the claw-like portions 160 and the front end of the elastic member 154 is x, a distance between the base end of the insertion assisting tool 70 and the groove 162 is y, and a distance between the base end of the claw-like portions 160 and the front end of the mounting ring 156 is z, the distance relation is configured to satisfy z > y > x. With such a configuration of z > y > x, when the claw-like portions 160 are engaged with the groove 162, the elastic member 154 is pressed between the mounting ring 156 and the insertion assisting tool 70. In this way, a gap between the insertion assisting tool 70 and the tubular body 152 is sealed by the elastic member 154.

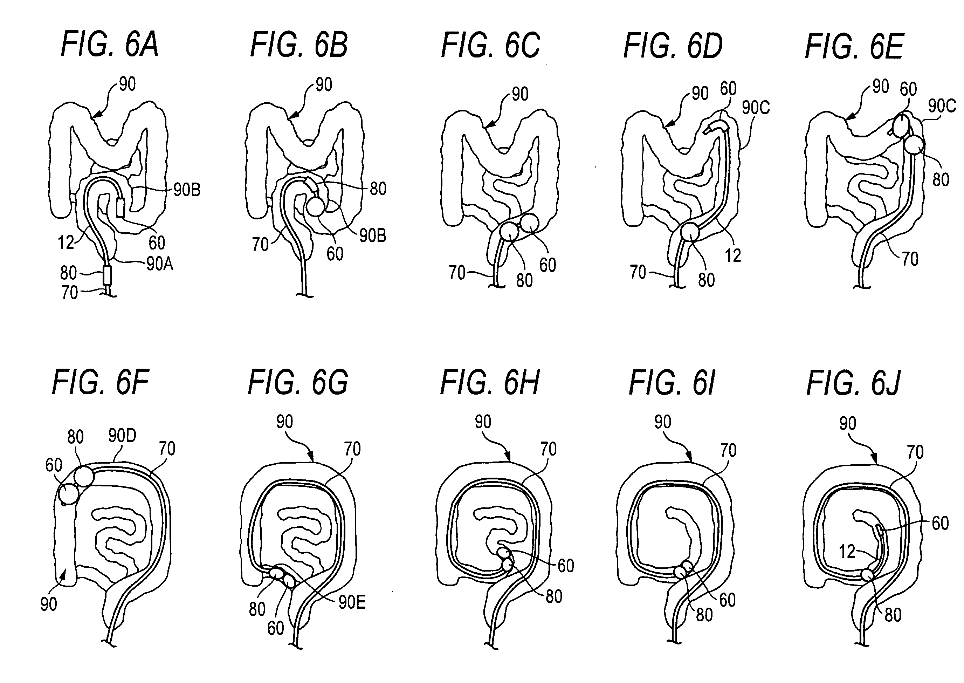

Next, an operation method of the endoscope apparatus configured in this manner will be described with reference to Figs. 6A to 6J and Figs. 7A and 7B.

First, the insertion section 12 is inserted into the seal member 150, and then inserted into the insertion assisting tool 70. Subsequently, as shown in Fig. 6A, the insertion section 12 is inserted from an anus 90A into an alimentary canal (large intestine) 90 (insertion operation). At this time, the first balloon 60 and the second balloon 80 are deflated. In addition, as shown in Fig. 7A, the seal member 150 is drawn up to the hand operation section 14.

Next, as shown in Fig. 6A, the first balloon 60 is inflated in a state where the front end of the insertion section 12 reaches an S-shaped colon 90B, and then the front end of the insertion section 12 is fixed to the alimentary canal 90 (fixation operation).

Subsequently, the insertion assisting tool 70 is inserted so as to follow the insertion section 12 (press operation). As shown in Fig. 6B, the front end of the insertion assisting tool 70 is inserted up to the vicinity of the first balloon 60, and then air is supplied to the second balloon 80 so as to be inflated. In this way, the second balloon 80 is fixed to the alimentary canal 90, so that the alimentary canal 90 is gripped by the insertion assisting tool 70 through the second balloon 80.

Next, as shown in Fig. 6C, the insertion assisting tool 70 is drawn so as to eliminate winding portion of the alimentary canal 90 (extraction operation).

Subsequently, the air is sucked from the first balloon 60 to deflate the first balloon 60. In addition, as shown in Fig. 6D, the insertion section 12 is inserted into the deeper portion of the alimentary canal 90 (for example, up to winding portion of the upper portion of the descending colon 90C) (insertion operation). As described above, the fixation operation of inflating the first balloon 60 and the press operation of following the insertion section 12 by inserting the insertion assisting tool 70 are performed. Afterward, the extraction operation of inflating the second balloon 80 to grip the alimentary canal 90 is performed by use of the insertion assisting tool 70. In this way, as shown in Fig. 6E, the winding portion of the alimentary canal 90 is eliminated.

A series of such operations (the insertion operation, the fixation operation, the press operation, a grip operation, and the extraction operation) are repeatedly performed, so that the front end of the insertion section 12 can be gradually inserted into the deeper portion of the alimentary canal 90. Moreover, the winding portion of the alimentary canal 90 can be eliminated by use of the insertion assisting tool 70.

For example, in Fig. 6F, the front end of the insertion section 12 is inserted into the end portion of the transverse colon 90D and the winding portion of the alimentary canal 90 is eliminated by performing the extraction operation of the insertion assisting tool 70.

The front end of the insertion section 12 can be inserted into the deeper portion of the large intestine by performing such operations. At this time, the insertion assisting tool 70 is shaped into a half loop, so that the insertion section 12 can be thereby inserted into the deeper portion of the alimentary canal 90. Therefore, the deeper portion of the alimentary canal 90 can be observed and treated by the endoscope 10.

However, if the insertion operation and the extraction operation of the insertion section 12 are repeatedly performed by using the insertion assisting tool 70 in the state where the insertion section 12 is inserted into the alimentary canal 90, the body fluid may flow backward beyond the gap between the inner circumferential surface of the insertion assisting tool 70 and the outer circumferential surface of the insertion section 12, and thus the body fluid may leak from the base end of the insertion assisting tool 70. In particular, when the insertion section 12 is inserted into the deep portion of the alimentary canal 90, resistance of the insertion operation and the extraction operation increases, and thus a leaking body fluid may increase.

In order to solve such a problem, after the insertion section 12 is inserted to some extent, the seal member 150 is mounted on the base end of the insertion assisting tool 70. That is, the tubular body 152 of the seal member 150 is gripped to be moved along the insertion section 12 to the front end side of the insertion section 12. Subsequently, the tubular body 152 is pushed into the gripping section 72 of the insertion assisting tool 70, so that the claw-like portions 160 are engaged with the groove 162 of the gripping section 72. In this way, the seal member 150 is mounted on the gripping section 72 of the insertion assisting tool 70. At this time, as shown in Fig. 7B, a size between the claw-like portions 160 and the side surface of the front end of the elastic member 154 is slightly shorter than that of the mount portion of the gripping section 72 of the insertion assisting tool 70. Accordingly, since the elastic member 154 is tightly pressed by the mounting ring 156 and the gripping section 72, the gap between the insertion assisting tool 70 and the seal member 150 is sealed. Moreover, since the cylindrical portion 154B of the elastic member 154 contacts the outer circumferential surface of the insertion section 12, a gap between the insertion section 12 and the elastic member 154 of the seal member 150 is sealed. In this way, since the gap between the insertion section 12 and the insertion assisting tool 70 is reliably sealed by the seal member 150, it is possible to reliably prevent the body fluid or the like from flowing backward.

According to this embodiment, the seal member 150 has a cylindrical portion 154B with a uniform diameter, so that the cylindrical portion 154B is closely contacted with the outer circumferential surface of the insertion section 12. Accordingly, the gap between the insertion section 12 and the insertion assisting tool 70 can be reliably sealed, fluid leakage can be prevented.

According to this embodiment, the elastic member 154 is supported by the hard tubular body 152. Accordingly, by gripping the tubular body 152, the elastic member 154 can be smoothly moved so as to slide without deformation of the elastic member 154. In addition, the tubular body 152 has the claw-like portions 160 to be fitted to the gripping section 72 of the insertion assisting tool 70. Accordingly, the mount portion on the insertion assisting tool 70 has no function of sealing the gap between the insertion section 12 and the insertion assisting tool 70 and the seal member 150 can be simply attached to or detached from the insertion assisting tool 70. That is, like JP-A-2005-312905, if the mounting operation on the insertion assisting tool 70 is performed by using an elastic force of a tube and the mount portion has the mounting function and the sealing function, it is difficult to perform the attaching and detaching operations. However, in this embodiment, since the tubular body 152 performs the mounting function on the insertion assisting tool 70 and the elastic member 154 performs the sealing function, it is possible to simply perform the attaching and detaching operations. Accordingly, the seal member 150 can be mounted on the insertion assisting tool 70 only if necessary. For example, when the insertion section 12 of the endoscope 10 is inserted into a shallow colon (for example, S-shaped colon) of the alimentary canal 90, the seal member 150 can be easily detached from the insertion assisting tool 70. In this way, since resistance at the time of inserting the insertion section 12 decreases, an operator can easily feel the resistance which the insertion section 12 receives from the alimentary canal 90, thereby improving operability. That is, when the seal member 150 is mounted on the insertion assisting tool 70, the friction between the seal member 150 and the insertion section 12 is large. Accordingly, when the resistance is large when the insertion section 12 is inserted, it is difficult for the operator to feel the resistance from the alimentary canal 90. For this reason, a problem arises in that the operability deteriorates. However, by separating the seal member 150, the operator can directly feel only the resistance from the alimentary canal 90 with hand. As a result, the above-described problem can be prevented.

According to this embodiment, the elastic member 154 has the hollow cylindrical shape. Accordingly, when the insertion section 12 is considerably incurved, the cylindrical portion 154B follows the insertion section 12, thereby normally maintaining adhesion between the cylindrical portion 154B and the insertion section 12. That is, as shown in Fig. 7B, the cylindrical portion 154B has the hollow portion. Accordingly even when the hand operation side of the insertion section 12 is inclined upward and the front end thereof is inclined downward, the shape of the cylindrical portion 154B can be deformed so that the front end side is squashed and the hand operation side is swollen in the lower portion and so that the front end is swollen and the hand operation side is squashed in the upper portion. Therefore, the cylindrical portion 154B can follow the motion of the insertion section 12. As a result, it is possible to reliably seal the gap between the insertion section 12 and the insertion assisting tool 70.

According to this embodiment, the seal member 150 is used to prevent the leakage of the body fluid. However, the invention is not limited thereto, but the seal member 150 can be also used to prevent leakage of the lubricant injected through the inlet port 78. When an X-ray radiopaque dye is injected through the inlet port 78, the seal member 150 may be used to prevent leakage of the radiopague dye.

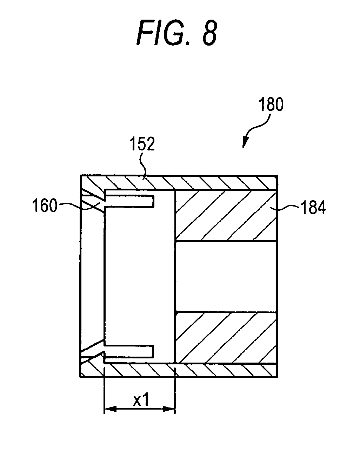

The configuration of the seal member 150 is not limited to the above-described embodiment. However, the seal member 150 may be configured in the manner that the seal member 154 has the cylindrical portion 154B with a uniform diameter, the elastic member 154 is supported by the hard tubular body 152, and furthermore the tubular body 152 may have a locking portion fitted to the insertion assisting tool 70. For example, in a seal member 180 shown in Fig. 8, an elastic member 184 is configured to have a cylindrical shape with a predetermined thickness. The elastic member 184 is supported by a hard tubular body 152 so as to be pressed in the inside of the tubular body 152 having a claw-like portion 160. In addition, the inner diameter of the elastic member 184 is slightly smaller than the outer diameter of the insertion section 12. The elastic member 184 contacts the outer circumferential surface of the insertion section 12. Even in the seal member 180 configured in this manner, the elastic member 184 can reliably seal the gap between the insertion section 12 and the insertion assisting tool 70. The seal member 180 can be easily attached to or detached from the insertion assisting tool 70 by engaging the claw-like portion 160 of the tubular body 152 having a groove 162 of the insertion assisting tool 70. In the seal member 180, "a distance x1 between the base end of the claw-like portion 160 and the front end of the elastic member 184" is slightly smaller than "a distance y between the base end of the insertion assisting tool 70 and the base end of the groove 162 (see Fig. 7A). In this case, the elastic member 184 closely contacts the entire surface of the base end of the assisting tool 70. Accordingly, it is possible to seal the gap between the insertion assisting tool 70 and the seal member 180.

The endoscope with two balloons according to the above-described embodiment has been described. However, the invention is not limited thereto, but can be applied to a case where the insertion assisting tool 70 is used without the second balloon 80 or a case where the endoscope 10 is used without the first balloon 60.

According to the invention, since the seal member can follow the insertion section in a state where an insertion section is inserted into a seal member, the seal member can be mounted on an insertion assisting tool only if necessary, thereby preventing leakage of a body fluid or the like and also improving operability.

The entire disclosure of each and every foreign patent application from which the benefit of foreign priority has been claimed in the present application is incorporated herein by reference, as if fully set forth.

## Claims

1. An endoscope apparatus comprising:
an endoscope including an insertion section;
a cylindrical insertion assisting tool that allows the insertion section to be inserted to assist insertion of the insertion section into a body cavity; and
a ring-shaped seal member that is supported so as to slide on the insertion section with the insertion section inserted,
wherein the seal member is detachably mounted on a base end of the insertion assisting tool to seal a gap between the insertion assisting tool and the insertion section.

2. The endoscope apparatus according to Claim 1, wherein the seal member comprises:
an elastic member including a cylindrical portion having a uniform diameter, an inner circumferential surface of the cylindrical portion being to closely contact an outer circumferential surface of the insertion section; and
a ring-shaped tubular body of a material harder than that of the elastic member that supports the elastic member and is detachably fitted to the base end of the insertion assisting tool.

3. The endoscope apparatus according to Claim 2,
wherein when the tubular body is fitted to the base end of the insertion assisting tool, the elastic member closely contacts the base end of the insertion assisting tool.

4. The endoscope apparatus according to Claim 2,
wherein the tubular body comprises a locking portion protruding from the inner circumferential surface of the tubular body, and
wherein the locking portion is fitted to the base end of the insertion assisting tool.

5. The endoscope apparatus according to Claim 2,
wherein the seal member further comprises a mounting ring having a diameter larger than that of the elastic member and having a length in an axial direction shorter than that of the elastic member, and
wherein both ends of the elastic member are turned over in a state where the elastic member is inserted into the mounting ring and adhered to an outer circumferential surface of the mounting ring, so that the mounting ring is inserted into the tubular body.
